# EUROPEAN PATENT APPLICATION

(11) **EP 0 596 215 A1**
(43) Date of publication of application: **11.05.1994**
(21) Application number: 93114247.5
(22) Date of filing: 06.09.1993
(51) Int. Cl.: A61L 15/28, A61F 13/02

(54) **Applying material for protecting wound surface**

(30) Priority: 02.11.1992 JP 317800/92; 02.11.1992 JP 317712/92; 05.11.1992 JP 352778/92; 05.11.1992 JP 352777/92
(71) Applicant: SUNFIVE COMPANY LTD, Tottori-shi, Tottori (JP); KANAE COMPANY LTD, Osaka-shi, Osaka (JP)
(72) Inventor: Ohta, Hisato, Sunfive Company Ltd., Tottori-shi, Tottori (JP); Takaishi, Kazuki, Kanae Company Ltd., Osaka-shi, Osaka (JP); Uetani, Noriko, Kanae Company Ltd., Osaka-shi, Osaka (JP)
(74) Representative: Türk, Gille, Hrabal, Leifert

(57) **Abstract**

An applying material (1) for protecting wound surface comprising film of the water soluble polymer (3), powdered chitin (2) coated on said film, body fluid absorbing layer (4), such as, compressed cotton, cellulose sponge and non-woven texture provided on the back surface of said film, tape material (6) for adhering adhered on said body fluid absorbing layer by adhesive (5).

## Description

### Background of the Invention

### Field of the Invention

The present invention relates to an applying material for protecting wound surface which remarkably improves the defect of the conventional goods by using the chitin powder together with water soluble polymer film which is dissolved by blood. It is already proved that chitin has effect of treating the wound and stopping blood.

### Prior art

Many materials are produced on a commercial scale, used and proposed as the protective material for applying on the wound surface. However, the conventional materials are cloth or film, thereby the tearing off from the wound surface is very difficult due to combining with granulation tissue after comparative long time using. If tearing off, the wound surface is undergone a bad influence.

Further, wound surface protective materials in which the chitin is used are suggested in Japanese patent applications No. 60-85169 and 60-23608 (publication No. 62-97557), it is proved that the chitin has the effect of stopping blood. The chitin is a kind of amino sugar polymer and is included in the shell of the Insect, the Crustacean and the Mollusca, and further in the shell of eggs of animals and birds. However, these conventional materials are made by forming the chitin in fiber form, and in which the pore volume corresponds the ooze out liquid volume from the wound surface. It has high adhesivity to the wound surface. It can be naturally teared in case of long time use, but it has disadvantages that tearing off gives pain and wound surface is made worse by adhesivity in case of short time use as the first-aid plaster.

### Summary of the Invention

The object of the present invention is to provide the wound surface protective material, in which the wound treatment effect and blood stopping effect are exibited during applying at wound surface and only chitin remains on the wound surface when the tearing off without pain and aggravation of the wound.

### Brief Description of the Drawings

Fig. 1 is enlarged sectional view of the first embodiment of the wound surface protective material according to the present invention,
Fig. 2 is same view of the improved embodiment of Fig. 1,
Fig. 3 is same view of the further improved embodiment of Fig. 1,
Fig. 4 is enlarged sectional view of the second embodiment of the wound surface protective material according to the present invention,
Fig. 5 is same view of the improved embodiment of Fig. 4,
Fig. 6 is same view of the further improved embodiment of Fig. 4,
Fig. 7 is enlarged sectional view of the third embodiment of the wound surface protective material according to the present invention,
Fig. 8 is enlarged sectional view of the forth embodiment of the wound surface protective material according to the present invention,

### Detailed Description of the Invention

Applying material of the present invention is made of film on which the powdered chitin is coated, or film formed from dispersion liquid including powdered chitin or a net on which the powdered chitin is adhered.

The invention is detailedly explained based on the following embodiments shown in drawings.

### Embodiment 1

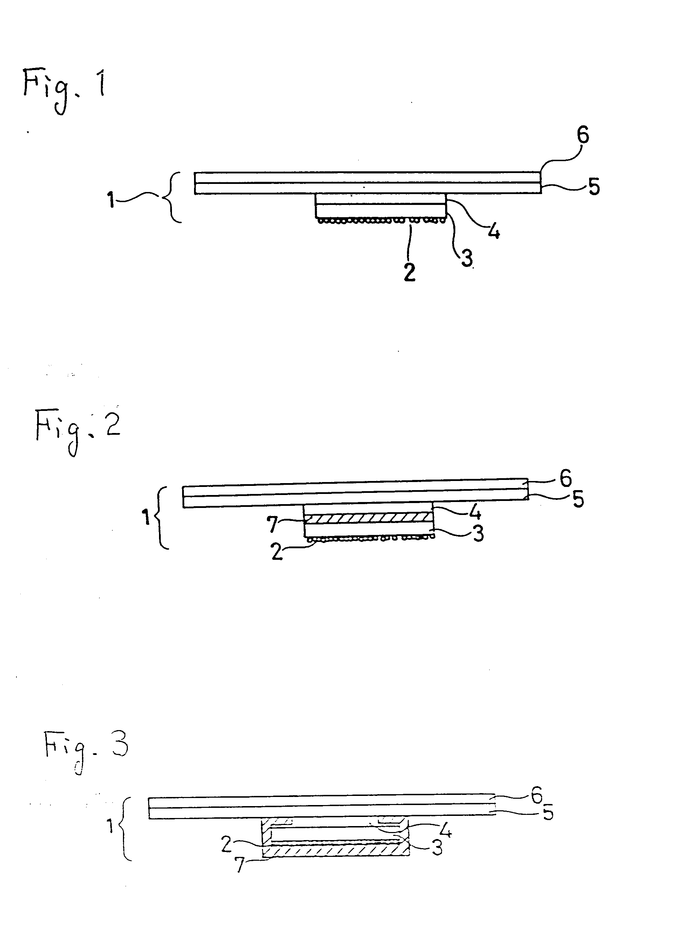

Fig. 1 is a sectional view of the first embodiment of the present invention (1). At first powdered chitin (2) is coated on the water soluble polymer film (3). This coated surface is an applying surface of the protective material (1). Water soluble polymer using in this embodiment is natural neutral multi-saccharide, is quickly dissolved in cold and hot water, and has low viscosity compared with other multi saccharide, and does not easily deteriorate, further has superior adhesivity, coating property and membrane forming property. This natural neutral multi saccharide using in this embodiment is Pluran manufactured by Hayashibara Co., Ltd.

Pluran film (3) is made by coating Pluran solution on the plastic film, for example, PET (polyethylene telephtalate) and drying. In the present invention, before Pluran is completely dried, powdered chitin is uniformly coated by spraying and the like, and the surface is dried.

In this embodiment, the chitin can be coated in the powdered form. Further in this case of the pluran film which pluran is dried, after applying the steam on the pluran surface powdered chitin can be coated to be applying surface.

Powdered chitin (2) is coated on the plastic film and dried, and Pluran film (3) is made by tearing the plastic film, such as PET off. Body fluid absorbing layer (4), such as compressed cotton, cellulose sponge, absorbing non woven fabric is provided on the hack surface of Pluran film (3). Further, sheet form tape material (6) is adhered on the back surface of the body fluid absorbing layer (4) by acrylic or siliconic and the like adhesive layer (5).

In order to protect Pluran layer (3) and the chitin powder layer (2) and to accelarate to tear the applying material (1) off, it is preferable that water insoluble net such as polyethlene net is provided between Pluran film (3) and body fluid absorbing layer (4) (Fig. 2), and/or outer of kichin powder layer (2) (Fig. 3).

One of the characteristics of this wound surface protective applying material is that chitin is existed in powder form. Further, since water soluble polymer (blood soluble) is used as binder and body fluid absorbing material is provided, each effect is synergistically shown. And in case of tearing off the protective material, only powdered chitin remains on the wound surface, thereby the wound surface is not injured and tearing is possible without pain.

### Example 2

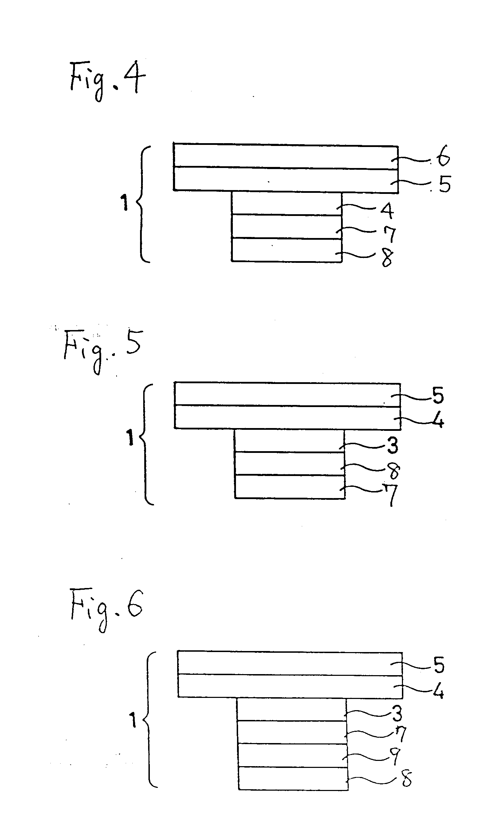

Fig. 4 is sectional view of the other embodiment of the applying material for protecting wound surface (1). Powdered chitin is uniformly distributed in the solution of water soluble polymer (for example, Pluran manufactured by Hayashibara Co. Ltd), and the liquid is formed in film form (8). Pluran is same as the embodiment shown in Fig. 1. The film is used as applying part of the applying material for protecting wound surface.

The effect of chitin powder is proved as mentioned above, in this embodiment, body fluid absorbing material layer (4) such as compressed cotton, cellulose sponge or non-woven fabric is provided on the back surface of Pluran film (2) in which the chitin powder is mixed and dispersed, and further the tape material for adhering to the skin is adhered on the back surface of the body fluid absorbing material layer (4) by acrylic or silicone adhesive layer and the like (5).

In order to protect Pluran film (8) in which the chitin powder is mixed and dispersed and to accelarate to tear the applying material (1) off, it is preferable that water insoluble net (7) is provided between pluran film (8) and body fluid absorbing layer (4) (Fig. 4, Fig. 5), and/or outer of Pluran film (8) (Fig. 6).

In the water soluble film including chitin, the quantity of chitin powder mixed is less than 50% by weight in dry condition. If the quantity is too much, the strength of the film including chitin powder may deteriorate. In this case, in order to reinforce the film, Pluran film including no chitin powder (9) may be provided as shown in Fig. 6.

One of the characteristics of this wound surface protective applying material is that chitin is formed in powder form. Further, since water soluble polymer (blood soluble) is used as binder and body fluid absorbing material is provided, each effect is synergistically shown. And in case of tearing off the protective material, only powdered chitin remains on the wound surface, thereby the wound surface is not injured and tearing is possible without pain.

### Example 3

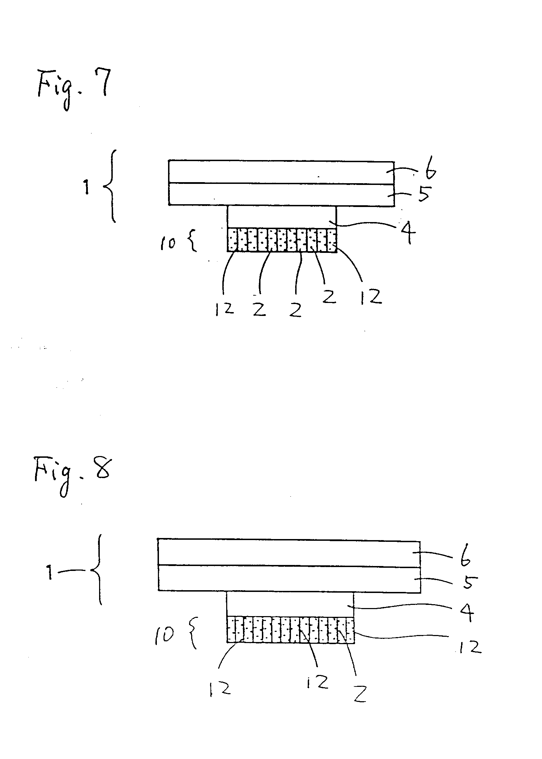

Fig. 7 is a sectional view of other embodiment of the applying material for protecting wound surface according to the present invention. Powdered chitin is uniformly dispersed in the solution of water soluble polymer (Pluran manufactured by Hayashibara Co. Ltd), and a net (12) is soaked in the liquid and dried. This net having powdered chitin (10) is an applying surface of the protective material. Body fluid absorbing material (4), such as compressed cotton, cellulose sponge or non-woven fabric is provided on the back surface. Further the tape material (6) for adhering to the skin is adhered on the back surface of the body fluid absorbing material layer (4) by acrylic or silicone adhesive layer and the like (5).

Knitting, texture or non-woven fabric using natural or synthetic fiber (Polyethylene, polyamide, cotton, rayon and the like) can be used as a net to be fixed powdered chitin (12). When fixing by soaking is difficult because of high shedding property of the net (12), the viscosity of the water soluble polymer may be made higher and the polymer liquid may be pushed out or coated on the net and dried.

One of the characteristics of this wound surface protective applying material is that chitin powder directly contacts the wound surface in dispersion state to improve the treatment effect. Further, since the quantity of Pluran is reduced by using the net, the flexibility of applying material can be maintained. In this embodiment, since chitin powder is dispersed in the water soluble binder which is dissolved by body fluid and is fixed to the net, the powder is not dropped in the circulation of the goods. And when the applying material is applied to the wound surface the hinder is dissolved by body fluid, thereby it is possible to tear off without pain. The quantity of Pluran can be saved since the flexible net is used.

### Example 4

Fig. 8 is a sectional view of other embodiment of the applying material for protecting wound surface according to the present invention. Powdered chitin is uniformly dispersed in the water or the solution which has less surface tension than water, such as, ethanol and isopropyl alcohol. And a proper quantity, that is, less than 20% of powdered chitin (preferably 1 to 10%) , of water holding agent, such as the solution in which water soluble and non-volatile compound (glycerine and the like) is dissolved, is coated on the net by gravure coating or reverse coating, and dried. The net on which chitin powder is fixed (10) is applying surface of the applying material.

As mentioned above, powdered chitin is fixed on the net by coating and drying, but it has no crack in it and initial applying condition maintains after drying by using water holding agent. The body fluid absorbing material layer (4), such as compressed cotton, cellulose sponge, non-woven fabric is adhered on the back surface of the net on which chitin powder fixed (10), further the tape material (6) is adhered on the body fluid absorbing material layer by acrylic or silicone adhesive (5).

Knitting, texture or non-woven fabric using natural or synthetic fiber (Polyethylene, polyamide, cotton, rayon and the like) can be used as a net to be fixed powdered chitin (12).

The solvent of dispersion liquid which is coated on the net (12) or in which the net is dipped may be water, but the solution which has less surface tension than water, such as, ethanol or isopropyl alcohol is preferably. By using such a liquid, the dispersion and the coating are more uniform, thereby adhesivity is improved. In this embodiment, if the water holding agent such as glycerine is not used, caoting on the net is possible, but it prevents the chitin from dropping after drying.

In this embodiment, the net on which chitin powder dispersed uniformly together with water holding agent in water or low surface tension solution is coated and dried is an applying surface of the applying material for protecting wound surface. Thereby, chitin is fixed without other binder, but chitin powder is not dropped in the circulation of the goods by the effect of water holding agent. When applying on the wound surface, chitin powder easily contacts and moves to wound part, and when the tearing off without pain and aggravation of the wound.

## Claims

1. An applying material for protecting wound surface comprising;
film of the water soluble polymer,
powdered chitin coated on said film,
body fluid absorbing layer, such as, compressed cotton, cellulose sponge and non-woven texture provided on the back surface of said film,
tape material for adhering adhered on said body fluid absorbing layer by adhesive.

2. An applying material for protecting wound surface set forth in claim 1, wherein said water soluble polymer film is made by coating the water soluble polymer solution on plastic film and spraying powdered chitin on it before drying the polymer.

3. An applying material for protecting wound surface set forth in claim 1, wherein said water soluble polymer film is made by steaming the film of the water soluble polymer and coating the powdered chitin before drying the steam.

4. An applying material for protecting wound surface set forth in claim 1, wherein water insoluble net is provided between water soluble polymer film and body fluid absorbing layer, and/or outer of chitin powder layer.

5. An applying material for protecting wound surface comprising;
film of the water soluble polymer manufactured by dispersing uniformly powdered chitin in the water soluble polymer solution,
body fluid absorbing layer, such as, compressed cotton, cellulose sponge and non-woven texture provided on the back surface of said film,
tape material for adhering adhered on said body fluid absorbing layer by adhesive.

6. An applying material for protecting wound surface set forth in claim 5, wherein water insoluble net is provided between Pluran film (3) and body fluid absorbing layer (4) , and/or outer of chitin powder layer (2).

7. An applying material for protecting wound surface set forth in claim 5, wherein water soluble polymer film is provided inside the chitin powder layer.

8. An applying material for protecting wound surface comprising;
a net which is soaked in the solution of water soluble polymer in which powdered chitin is uniformly dispersed; and is dried,
body fluid absorbing layer, such as, compressed cotton, cellulose sponge and non-woven texture provided on the back surface of said film,
tape material for adhering adhered on said body fluid absorbing layer by adhesive.

9. An applying material for protecting wound surface set forth in claim 8, wherein knitting; texture or non-woven fabric using natural or synthetic fiber (Polyethylene, polyamide, cotton, rayon and the like) can be used as a net.

10. An applying material for protecting wound surface set forth in claim 8, wherein the viscosity of the water soluble polymer may be made higher and the polymer liquid may be pushed out or coated on the net and dried, when fixing by soaking is difficult because of high shedding property of the net (12).

11. An applying material for protecting wound surface comprising;
a net which is soaked in the the solurion which is water or the solution which has less surface tension than water, and is added water holding agent, and dried,
body fluid absorbing layer, such as, compressed cotton, cellulose sponge and non-woven texture provided on the back surface of said film,
tape material for adhering adhered on said body fluid absorbing layer by adhesive.

12. An applying material for protecting wound surface comprising;
a net on which the the solurion which is water or the solution which has less surface tension than water, and is added water holding agent is coated and dried,
body fluid absorbing layer, such as, compressed cotton, cellulose sponge and non-woven texture provided on the back surface of said film,
tape material for adhering adhered on said body fluid absorbing layer by adhesive.

13. An applying material for protecting wound surface set forth in claimll or 12, wherein low molecule alcohol such as ethanol, isopropyl alohol can be used as the solution which has less surface tension than water.

14. An applying material for protecting wound surface set forth in claimll or 12, wherein water soluble and non-volatile compound such as glycerine can be used as water holding agent.
